# EUROPEAN PATENT APPLICATION

(11) **EP 4 603 473 A1**
(43) Date of publication of application: **20.08.2025**
(21) Application number: 23891956.7
(22) Date of filing: 14.11.2023
(51) Int. Cl.: C07C 51/487, C07C 51/43, C07C 63/26, C08G 63/16, C08J 11/24

(54) **METHOD FOR CHEMICAL-MECHANICAL DEPOLYMERIZING POLYESTER**

(30) Priority: 15.11.2022 KR 20220152963; 25.09.2023 KR 20230128165
(71) Applicant: Kolon Industries, Inc., Seoul 07793 (KR)
(72) Inventor: GYOUNG, Chung-Hyoun, Seoul 07793 (KR); LEE, Won Hee, Seoul 07793 (KR); LEE, Dong Eun, Seoul 07793 (KR); PARK, Il-Ho, Seoul 07793 (KR)
(74) Representative: Ter Meer Steinmeister & Partner
(86) International application number: PCT/KR2023/018207
(87) International publication number: WO 2024/106889

(57) **Abstract**

Provided is a method for chemical-mechanical depolymerizing polyester, which includes: pulverizing polyester in the presence of a decomposer; generating heat; depolymerizing the polyester into a dibasic acid and an alkylene glycol; and separating the alkylene glycol by vaporizing it using the heat

## Description

### [Technical Field]

The present disclosure relates to a method for chemical-mechanical depolymerizing polyester.

### [Background Art]

A polyester represented by polyethylene terephthalate (PET) has excellent chemical stability and thus is used for fibers, fabrics, clothes, films, sheets, beverage bottles, or the like.

As usage of the polyester rapidly increases, various method for recovering and reusing a waste thereof are reviewed. As one of them, a so-called chemical recycling method is performed by depolymerizing the waste of the polyester and the like to convert it into monomers and recover it as the monomers, and use the monomers as raw materials to repolymerize them into recycled polyester such as polyethylene terephthalate and the like.

This chemical recycling of the polyester, which may separate and secure not much different quality of the recycled polyester from that of a virgin one as raw materials, is expected to realize reuse of resources.

There are three main methods of depolymerization of polyester into monomers: a hydrolysis method using water as a solvent, an alcoholysis method using an alcohol as a solvent, and a glycolysis method using a glycol as a solvent.

The hydrolysis method may be, for example, to decompose polyester into terephthalic acid and ethylene glycol by reacting a polyethylene terephthalate melt with water, and then reacting it with ammonium hydroxide (refer to Patent Document 1). This method has an advantage of using neither a glycol nor alcohol for the reactions, which proceed under high-pressure conditions, and thus requires a special high-pressure reactor.

The alcoholysis method is, for example, to depolymerize polyester by heating the polyester in an alcohol solvent (if necessary, by adding a catalyst), (refer to Patent Documents 2 and 3). This method, when polyethylene terephthalate is depolymerized by using, for example, methanol as a solvent, has an advantage of directly producing dimethylterephthalate (DMT), which is a useful and easy-to-handle monomer, by the depolymerization reaction, which proceeds relatively fast. However, alcohol, when used as a solvent, has a low boiling point and requires pressurization for a reaction to proceed (for example, reaction in supercritical or subcritical methanol), which requires a special high-pressure reactor.

The glycolysis method is to produce bis(β-hydroxyalkyl) terephthalate and ethylene glycol by depolymerizing polyester by heating with a depolymerization catalyst such as sodium carbonate and the like in an alkylene glycol solvent (Patent Documents 4 and 5). For example, when the ethylene glycol is used as the solvent, the bis(β-hydroxyethyl) terephthalate (BHET) is produced through the depolymerization reaction, and dimethylterephthalate (DMT) may be recovered by adding methanol thereto under a transesterification catalyst to carry out a transesterification reaction. The glycolysis method may carry out the reactions under a normal pressure but requires a relatively longer reaction time, which needs to be shortened, and has a problem of deteriorating glycol of the solvent by heating for a long time.

In addition, these known methods of depolymerizing polyester need a reaction time of at least several hours and thus have difficulties in processing a large amount of polyester waste, and also conduct the reaction under high temperature or high-pressure conditions and thus require special equipment capable of withstanding these conditions. Therefore, commercialization therefor has not yet been achieved.

### [Prior Art Documents]

### [Patent Documents]

(Patent Document 1) Japanese Patent Laid-Open Publication No. 2003-527363 (Publication Date: 2003.09.16)
(Patent Document 2) Japanese Patent Laid-Open Publication No. 1999-100336 (Publication Date: 1999.04.13)
(Patent Document 3) Japanese Patent Laid-Open Publication No. 2003-300916 (Publication Date: 2003.10.21)
(Patent Document 4) Japanese Patent Laid-Open Publication No. 2002-167468 (Publication Date: 2002.06.11)
(Patent Document 5) Japanese Patent Laid-Open Publication No. 2004-300115 (Publication Date: 2004.10.28)

### [Disclosure]

### [Technical Problem]

One aspect of the present disclosure may provide a method for chemical-mechanical depolymerizing polyester, which may shorten the process and reduce heat energy to secure an environmental advantage and reduce a manufacturing cost.

Another aspect of the present disclosure may provide a composition for polymerizing recycled polyester according to the method for chemical-mechanical depolymerizing polyester.

Still another aspect of the present disclosure may provide recycled polyester obtained by the method for chemical-mechanical depolymerizing polyester and prepared by using dibasic acid and alkylene glycol.

### [Technical Solution]

According to one aspect, method for chemical-mechanical depolymerizing polyester includes pulverizing polyester in the presence of a degradation agent, generating heat, and depolymerizing the polyester into dibasic acid and alkylene glycol; and separating the alkylene glycol by vaporizing it using the heat.

The depolymerizing may be performed in a pulverizing reactor including a rotating disk.

The pulverizing reactor may further include blades attached to the disk.

The pulverizing reactor may further include baffles on the inner wall.

The pulverizing reactor may further include an outlet for discharging vaporized alkylene glycol.

**The** heat may include pulverizing heat generated by pulverizing the polyester, frictional heat generated by friction of the polyester with the pulverizing reactor, or both.

**The** depolymerizing may be a solvent-free reaction that proceeds without adding a solvent.

**The** depolymerizing may be performed without applying additional heat from the outside other than the above heat.

**The** method for chemical-mechanical depolymerizing polyester may not further include the process of pulverizing, crushing, or cutting the polyester before or after depolymerizing.

The depolymerizing may be performed by rotating the disk at 500 rpm to 5000 rpm.

The internal temperature of the pulverizing reactor may be 130 °C to 300 °C due to heat.

The depolymerizing may be performed for 5 and 120 minutes.

The degradation agent may include an alkali, an acid, a salt thereof, a mono-alcohol, a polyhydric alcohol, or a mixture thereof.

The alkali may include sodium hydroxide, potassium hydroxide, lithium hydroxide, ammonia, or a mixture thereof.

The acid may include hydrochloric acid, nitric acid, sulfuric acid, carbonic acid, phosphoric acid, acetic acid, hypochlorous acid (HClO), or a mixture thereof.

The salt may include carbonate, hydrogen carbonate, phosphate, sulfate, sulfite, nitrate, silicate, hypochlorite, formate, acetate, citrate, oxalate, or a mixture thereof.

The mono-alcohol may include methanol, ethanol, propanol, butanol, or a mixture thereof.

The polyhydric alcohol may include ethylene glycol, n-propylene glycol, isopropylene glycol, diethylene glycol, polyethylene glycol, triethylene glycol, dipropylene glycol, 1,3-butanediol, 1,4-butanediol, glycerine, benzyl alcohol, polypropylene glycol, pentaerythritol, trimethylolpropane, or a mixture thereof.

The degradation agent may be in solid state or in aqueous solution.

The degradation agent may be added at 0.75 mol to 3.0 mol per 1.0 mol of a dibasic acid in the polyester.

In the method for chemical-mechanical depolymerizing polyester, alkylene glycol may be separated and the remaining dibasic acid may be obtained in the form of a solid-phase salt.

The method for chemical-mechanical depolymerizing polyester may further include additionally separating the remaining alkylene glycol from the solid-phase dibasic acid salt.

In the method for chemical-mechanical depolymerizing polyester, the obtained solid-phase dibasic acid salt is dissolved in water, an aqueous solution of the dibasic acid salt is neutralized with acid to precipitate dibasic acid crystals, and the dibasic acid crystals are separated from the precipitated product by solid-liquid separation.

The method for chemical-mechanical depolymerizing polyester may remove impurities from an aqueous solution of dibasic acid salt.

The method for chemical-mechanical depolymerizing polyester may recrystallize dibasic acid crystals.

A composition for polymerizing recycled polyester according to another aspect includes a dibasic acid and an alkylene glycol, obtained by the method for chemical-mechanical depolymerizing polyester.

A recycled polyester according to another aspect is produced using dibasic acid and alkylene glycol, obtained by the method for chemical-mechanical depolymerizing polyester.

A color of the recycled polyester may have an L-value of 45 or higher.

### [Advantageous Effects]

The method for chemical-mechanical depolymerizing polyester according to an aspect of the present invention, which requires no additional process such as washing, pulverizing, processing, or the like before adding a waste collected from industries or common households to a chemical process, is not limited to types of the waste, and needs neither a large amount of additional solvent nor a separate heat source for a reaction to proceed with a simple device configuration and a low investment cost and also, is environmentally friendly due to no treatment and disposal of the solvent.

### [Description of the Drawings]

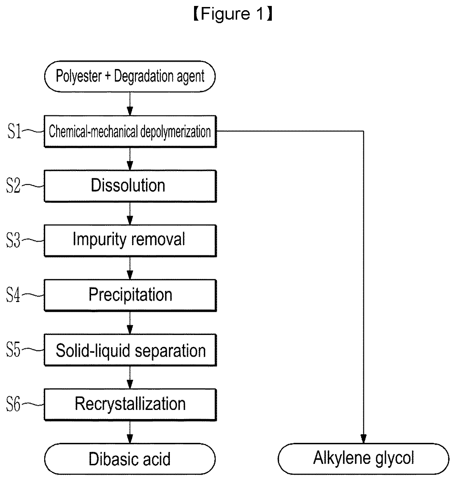

FIG. 1 is a process flowchart showing the method for chemical-mechanical depolymerizing polyester.

### [Best Mode]

Advantages and characteristics of this disclosure, and a method for achieving the same, will become evident referring to the following exemplary embodiments together with the drawings attached hereto. However, the embodiments should not be construed as being limited to the embodiments set forth herein. If not defined otherwise, all terms (including technical and scientific terms) in the specification may be defined as commonly understood by one skilled in the art. The terms defined in a generally-used dictionary may not be interpreted ideally or exaggeratedly unless clearly defined.

In addition, unless explicitly described to the contrary, the word "comprise," and variations such as "comprises" or "comprising," will be understood to imply the inclusion of stated elements but not the exclusion of any other elements. Further, the singular includes the plural unless mentioned otherwise.

FIG. 1 is a process flowchart showing the method for chemical-mechanical depolymerizing polyester according to one aspect. Referring to FIG. 1, the method for chemical-mechanical depolymerizing polyester is described.

The method for chemical-mechanical depolymerizing polyester is to pulverize polyester under the presence of a degradation agent to generate heat and depolymerize the polyester (S1).

The polyester may be made by polymerizing a dibasic acid with alkylene glycol, and may be polyethylene terephthalate (PET), polybutylene terephthalate, and polyethylene naphthalate, or polycaprolactone made by polymerizing caprolactone. By depolymerizing these polyesters, the dibasic acid, alkylene glycol, or caprolactone may be additionally recovered as monomers.

The alkylene glycols that can be obtained by depolymerizing polyester may include, for example, ethylene glycol, diethylene glycol, triethylene glycol, polyethylene glycol, propylene glycol, and dipropylene glycol, 1,3-butanediol, 1,4-butanediol, 3-methyl-1,5-pentanediol, 1,6-hexanediol, 1,9-nonanediol, neopentyl glycol, polytetramethylene glycol, 1,4-cyclohexanediol, 1,4-benzene diol, and the like. For example, if the polyester is polyethylene terephthalate, ethylene glycol can be recovered as an alkylene glycol, and if the polyester is polybutylene terephthalate, butylene glycol can be recovered as a monomer.

The dibasic acid that can be obtained by depolymerizing polyester may include, for example, terephthalic acid, phthalic acid (ortho), isophthalic acid, dibromoisophthalic acid, sodium sulfoisophthalate, phenylenedioxy dicarboxylic acid, 4,4'-diphenyldicarboxylic acid, 4,4'-diphenylether dicarboxylic acid, 4,4'-diphenylketone dicarboxylic acid, 4,4'-diphenoxyethanedicarbonic acid, 4,4'-diphenylsulfonic dicarbonic acid, and an aromatic dicarboxylic acid such as 2,6-naphthalene dicarboxylic acid, trimellitic acid, and pyromellitic acid. Other dicarboxylic acid may include, for example, aliphatic ring dicarboxylic acid such as hexahydroterephthalic acid and hexahydroisophthalic acid, and an aliphatic dicarboxylic acid such as succinic acid, glutaric acid, adipic acid, pimelic acid, suberic acid, azelaic acid, sebacic acid, undecacarbonic acid, and dodecadic dicarboxylic acid.

As a raw material for polyester depolymerization, molded products including polyester, especially waste, may be used. The waste may be a residue and a defective product generated during the manufacture of molded products as well as a waste generated after using the molded products including polyester. For example, the waste may include used PET bottles, cups, strings, packing packs, etc., or burrs and sprues formed during their moldings, cups after vacuum molding, sheets after cutting, fibers, fabrics, clothes, films, sheets, etc.

Herein, in the polyester waste, a content of polyester may be 60 wt% to 100 wt% based on a total weight of the polyester waste. When the content of polyester is less than 60 wt%, the depolymerization process may obtain so much smaller an amount of subsidiary raw materials than those of by-reactants and non-recyclable separated and purified wastes, which is substantially uneconomical.

When the polyester waste is used as a feedstock for the depolymerization, the waste may be optionally washed before the depolymerization and pre-treated to remove contaminants attached to the waste, such as dyes, contents, soil, or the like.

In addition, since the method for chemical-mechanical depolymerizing polyester simultaneously involves depolymerization with pulverization, a polyester waste recovered therefrom may be depolymerized itself or into relatively large pieces but alternatively, mechanically cut, pulverized, or processed into appropriate sizes. The pulverization may be carried out by using a known suitable means, for example, a hammer mill etc. to pulverize the polyester waste into small pieces with a size of 2 mm to 8 mm and provide them for the depolymerization reaction.

In addition, if necessary, the polyester may be immersed in a solvent to extract dyes and the like or to separate lighter components than the solvent, or may be blown away by wind or sieved to recover ones with predetermined size, which are provided for the depolymerization reaction.

However, as mentioned above, the method for chemical-mechanical depolymerizing polyester pulverizes polyester and simultaneously, depolymerizes it by heat generated therefrom, which requires neither additional processes such as washing, pulverizing, processing, or the like for adding wastes collected from industries or common households to a chemical process nor additional processing (flake, popcorn) processes and uses all wastes in various forms as a feedstock.

The degradation agent may include an alkali, an acid, a salt thereof, a mono-alcohol, a polyhydric alcohol, or a mixture thereof. In addition, as described later, when the depolymerizing reaction proceeds as a solvent-free reaction, the degradation agent may be added in a solid state or an aqueous solution state.

For example, the alkali may include a hydroxide of an alkali metal or alkaline earth metal, ammonia, or a mixture thereof. The alkali metal may be a monovalent metal such as lithium, sodium, potassium, rubidium, or cesium, of which sodium or potassium is relatively inexpensive. The alkaline-earth metal may be beryllium, magnesium, calcium, strontium, barium, or radium. The hydroxide of an alkali metal may include sodium hydroxide, potassium hydroxide, or lithium hydroxide. Among them, sodium hydroxide has an excellent reaction rate and reaction yield when used in combination with ethylene glycol, etc.

The acid may be an organic acid or inorganic acid, for example hydrochloric acid, nitric acid, sulfuric acid, carbonic acid, phosphoric acid, acetic acid, hypochlorous acid (HClO), or a mixture thereof.

The salt may include an inorganic salt such as carbonate, a hydrogen carbonate salt, phosphate, sulfate, sulfite, nitrate, silicate, hypochlorite, an organic salt such as formate, acetate, citrate, oxalate, or a mixture thereof, and the salt may include, for example, sodium carbonate, sodium hydrogen carbonate, potassium carbonate, potassium hydrogen carbonate, trisodium phosphate (hydrate), sodium hypochlorite, or a mixture thereof.

The polyhydric alcohol may include ethylene glycol, n-propylene glycol, isopropylene glycol, diethylene glycol, polyethylene glycol, triethylene glycol, dipropylene glycol, 1,3-butanediol, 1,4-butanediol, glycerine, benzyl alcohol, polypropylene glycol, pentaerythritol, trimethylolpropane, or a mixture thereof. Among these polyhydric alcohols, ethylene glycol, n-propylene glycol, isopropylene glycol, diethylene glycol, polyethylene glycol, triethylene glycol, and glycerine, which have a high boiling point and are relatively reactive, may be used.

The mono-alcohol may include methanol, ethanol, propanol, butanol, or a mixture thereof, and may include an alkyl ether compound of the polyhydric alcohol such as the diol and triol described above, for example diethylene glycol monomethyl ether, benzyl alcohol, 2-ethyl hexanol, and the like.

Since the degradation agent reacts with both polyester and dye, the degradation agent may be added in an amount of 0.75 mol to 3.0 mol based on 1.0 mol of dibasic acid in the polyester, and for example, a monovalent degradation agent (e.g., sodium hydroxide) may be added in an amount of 1.5 mol to 3.0 mol, and a divalent degradation agent (e.g., calcium carbonate) may be added in an amount of 0.75 mol to 1.5 mol.

For example, when polyethylene terephthalate is decomposed by using sodium hydroxide as the degradation agent, since its reaction scheme is PET (polyethylene terephthalate) + 2NaOH → TPA salt (disodium terephthalate) + EG (ethylene glycol), 2 mol (420 g) of NaOH per 1 mol of TPA is required to decompose 1 kg of PET.

In addition, when the polyester feedstock is fibers, an amount of dyes added during the processing varies from 0.5 wt% to 20 wt% (based on the weight of fabric (o.w.f.)) of a weight of the polyester fibers, an amount of the degradation agent may be 2.0 mol or more to 1.0 mol of dibasic acid included in the polyester to fully decompose the dyes and the polyester fibers.

Since the method for chemical-mechanical depolymerizing polyester pulverizes the polyester under the presence of a degradation agent and simultaneously, depolymerizes it by using heat generated therefrom, which requires no high-pressure reactor, a device may be configured as a batch-type or continuous reactor. For example, the reactor is internally substituted with nitrogen (N₂) to suppress a side reaction after adding raw materials thereto, so that a reaction may proceed under the nitrogen (N₂) atmosphere during the entire process time.

The depolymerizing may be performed inside the pulverizing reactor including an equipment capable of pulverizing the polyester. For example, the pulverizing reactor may include a rotating disk at the bottom. The disk may pulverize the polyester through rotation, so that the polyester and the inner wall of the pulverizing reactor may be rubbed each other.

In order to further efficiently pulverize the polyester, the disk may have blades attached to the surface, for example, in the form of a mixer. In addition, the pulverizing reactor may further include baffles on the inner wall to increase friction heat generated by the friction between the polyester and the inner wall of the pulverizing reactor.

Inside the pulverizing reactor, pulverizing heat is generated by the pulverization of the polyester, while the friction heat is generated by the friction of the polyester with the inner wall of the pulverizing reactor. The generated heat causes a reaction of the polyester with the degradation agent, through which the polyester is depolymerized. For example, the heat may include the pulverizing heat, the friction heat, or both of them.

In this way, since the method for chemical-mechanical depolymerizing polyester may pulverize the polyester under the presence of the degradation agent and simultaneously, depolymerize the polyester by using the heat generated therefrom, the reaction may proceed as a solvent-free reaction without adding a large amount of a solvent and also, without applying additional heat in addition to the heat generated thereinside. For example, the method for chemical-mechanical depolymerizing polyester may be applied without adding a solvent such as water or ethylene glycol.

In addition, the method for chemical-mechanical depolymerizing polyester, which needs no separate heat control, no addition of a large amount of the solvent to increase uniformity of the reaction, or no separate heat source for the reaction, may be applied with a simple device configuration and a low investment cost and also, which requires neither treatment nor disposal of the solvent, may achieve an environmental advantage. Furthermore, dibasic acid, a depolymerization product, may be obtained as a salt in the form of paste, powder, or a pellet, which is easy to store and transport with a smaller volume.

For example, the depolymerizing may be performed by rotating the disk at 500 rpm to 5000 rpm, for example, 700 rpm to 2500 rpm, or 1000 rpm to 1200 rpm. When the disk has a rotation speed of less than 500 rpm during the depolymerizing, which brings about ineffective pulverization, the heat may be insufficiently generated by the pulverization and friction, but when the rotation speed is greater than 5000 rpm, the heat may be excessively generated by the pulverization and friction.

For example, the pulverizing reactor may have an internal temperature of 130 °C to 300 °C, for example 170 °C to 280 °C, or 200 °C to 250 °C due to the generated heat. When the internal temperature of the pulverizing reactor is less than 130 °C, efficiency of the depolymerization may be deteriorated, but when the internal temperature is greater than 300 °C, a side reactant of the depolymerization may occur.

The depolymerizing may be performed for 5 minutes to 120 minutes, 5 minutes to 60 minutes, or 10 minutes to 40 minutes. The depolymerization reaction time may be increased within a range of 120 minutes, as impurities including dyes of the polyester have a higher concentration, the polyester is processed in a larger amount, and the pulverizing reactor has a lower internal temperature.

On the other hand, the method for chemical-mechanical depolymerizing polyester may separate and recover alkylene glycol by gasifying it with the generated heat during the depolymerization.

The method for chemical-mechanical depolymerizing polyester may proceed as a solvent-free reaction by pulverizing polyester under the presence of a degradation agent and depolymerizing the polyester by using the heat generated therefrom and since the internal temperature of the pulverizing reactor may reach 300 °C at most by the pulverizing heat and/or friction heat, may separate and recover alkylene glycol produced therefrom by gasifying it during the depolymerization.

However, the present invention is not limited thereto, but in order to separate and recover the alkylene glycol by gasifying it, additional heat may be supplied from outside. In other words, the depolymerization of the polyester may be sufficiently carried out by the pulverizing heat and friction heat generated inside the pulverizing reactor, but the pulverizing heat and friction heat may not be enough to secure the gasification of the alkylene glycol. Herein, the additional heat may be supplied from outside.

On the contrary, a conventional alkali hydrolysis depolymerizing method is to depolymerize polyester in a reactor at a high temperature by adding a large amount of a depolymerizing solvent. Herein, required is a separate process of separating liquid-phased alkylene glycol and solid-phased dibasic acid salt from a depolymerizing product obtained during the depolymerization. In addition, the depolymerizing solvent and the polyester are heated to a reaction temperature, which consumes lots of energy. Furthermore, the used depolymerizing solvent may be separated from the product to be reused, but this may be controlled through a separate purification process, which incurs a lot of cost. In addition, side-reaction materials may be added depending on types of depolymerizing solvents used, which may bring about a problem of controlling impurities therefrom.

The method for chemical-mechanical depolymerizing polyester may recover the alkylene glycol during the depolymerizing process in an amount of greater than or equal to 0 wt% or less than or equal to 100 wt%, for example, 30 wt% to 95 wt%, or 50 wt% to 98 wt% based on a total weight of the alkylene glycol. When 0 wt% of the alkylene glycol is recovered during the depolymerization, since the alkylene glycol is discharged as a mixture with the produced dibasic acid, a separate process for separating it before dissolution is required, but when 100 wt% of the alkylene glycol is recovered, since the alkylene glycol is not mixed with the produced dibasic acid, the dibasic acid may be immediately dissolved to proceed to a next process.

In addition, the pulverizing reactor may further include an outlet for discharging the gasified alkylene glycol. The gasified alkylene glycol may be under a normal pressure condition and thus recovered by injecting nitrogen (N₂) thereinto to liquefy it with its gas flow in a condenser.

After the depolymerizing reaction, the dibasic acid remaining after separating the alkylene glycol may be obtained in the form of a solid salt.

However, the alkylene glycol may not be recovered at all, or a portion thereof may be recovered depending on the rotation speed of the disk of the pulverizing reactor, wherein the depolymerizing product may include a mixture of dibasic acid salt and residual alkylene glycol. The method for chemical-mechanical depolymerizing polyester optionally may further include a process of recovering alkylene glycol after separating the alkylene glycol and the dibasic acid salt from the depolymerizing product. For example, a method of separating the alkylene glycol produced by the depolymerization reaction from the degradation agent is not particularly limited in the present invention, but an appropriate method may be selected depending on a target compound, for example, a distillation concentration method. The distillation concentration may be performed by using a conventional distillation concentration device, for example, a vacuum distillation continuous device, a vacuum distillation batch device, and the like. Then, a solid-phase dibasic acid salt can be obtained through a drying process.

In addition, depending on the type of degradation agent used during depolymerization, it can be divided into hydrolysis method using alkali, alcoholysis method using alcohol, and glycolysis method using glycol, and the type of dibasic acid obtained accordingly also varies.

When the glycolysis method is used to depolymerize polyester, a product obtained therefrom may vary depending on the types of the degradation agents used in the depolymerization reaction. For example, when ethylene glycol is used as the degradation agent for the depolymerization of the polyethylene terephthalate, bis(β-hydroxyethyl) terephthalate (BHET) as monomers may be obtained, and when propylene glycol is used as the degradation agent for the depolymerization, bis(β-hydroxyethyl isopropyl)terephthalate (BHEPT) may be mainly obtained as monomers.

In addition, when benzyl alcohol and tripotassium phosphate are used as the degradation agent, an ester compound of an oligomer obtained therefrom has good solubility into chloroform and the like and thus may be efficiently recovered through solvent extraction. Furthermore, the ester compound of the dibasic acid or oligomer may be recovered by using devices for filtration, distillation, or the like.

The ester compound (e.g., BHET) of the dibasic acid or oligomer thus obtained may also be recovered as the methyl ester (e.g., DMT) of the dibasic acid or oligomer by a transesterification reaction with methanol.

The transesterification reaction may be performed by appropriately using, for example, a known method of transesterifying a depolymerization concentrate and methanol under a transesterification catalyst (an alkali metal compound, etc.) at 65 °C to 85 °C for 0.5 hours to 5 hours, and preparing a slurry in which solid DMT is dispersed in a mixed solution of methanol, alkylene glycol, and the like. In addition, a cake containing DMT may be separated by using a solid-liquid separator and the like, and then purified through distillation, recovering purified DMT.

On the other hand, when the hydrolysis method is used to depolymerize the polyester, dibasic acids or dibasic acid salts such as dialkali metal salts of oligomers may be produced, wherein these products may vary depending on types of alkali metals used in the depolymerization reaction. For example, when the sodium hydroxide is used as the degradation agent to depolymerize the polyethylene terephthalate, sodium terephthalate with ethylene glycol is produced.

Of these, dibasic acid salts (e.g., sodium terephthalate) are insoluble in alkylene glycol and form solid crystals, which can be easily separated from the solvent by a filtration method such as solid-liquid separation. Furthermore, alcohols attached to the obtained powdery crystals can be removed by washing with alcohols such as methanol or ethanol.

Subsequently, the crystals of the dibasic acid salt may be dissolved in water (S2), followed by a neutralization reaction with a mixture of acids to precipitate a dibasic acid (e.g., terephthalic acid) (S4). The precipitated dibasic acid can be recovered by separating water and solid-liquid using methods such as centrifugation (S5).

An amount of water added during the recovery process may be from 3 to 10 parts by weight based on 1 part by weight of the dibasic acid salt. In order to separate the dibasic acid therefrom, a content of acid added thereto may be equal to or more than that of alkali metals included in the dibasic salt by moles, wherein types of the acid may be inorganic acids such as hydrochloric acid, sulfuric acid, phosphoric acid, and the like which are strong acids with pH 2 or organic acids such as formic acid, acetic acid, oxalic acid, and the like, wherein the inorganic acid, particularly, hydrochloric acid or sulfuric acid, may be appropriate to reduce impurities in the produced monomers. The neutralization reaction may be performed at 20 °C to 85 °C and in general completed within 10 minutes to 5 hours.

On the other hand, the method for chemical-mechanical depolymerizing polyester may further optionally include removing impurities contained in polyester molded products or waste from an aqueous solution of dibasic acid (S3).

Means for removing the impurities are not particularly limited but may include any appropriate technique and device. For example, solids such as unreacted resins (polyethylene, polypropylene, polyvinyl chloride, etc.) other than polyester, and undissolved degradation agents such as alkali metals and the like, etc. may be removed by using a mesh or the like. In addition, the mesh does not remove dyes, fillers, and the like, which may be removed centrifugal separation, and filtration by an adsorbent such as activated carbon and the like, etc.

Since the obtained dibasic acid crystals show particle distribution characteristics of several micrometers to hundreds of micrometers, recrystallization of dibasic acid crystals with a commercially available particle size (100 µm or more) may be optionally included (S6). The recrystallization method is not particularly limited but may be, for example, performed at a high temperature under a high pressure after mixing the dibasic acid crystals with water.

The dibasic acid and the alkylene glycol obtained by the method for chemical-mechanical depolymerizing polyester may be reused as monomers for polymerizing recycled polyester. Specifically, a composition for polymerizing the recycled polyester including the dibasic acid and the alkylene glycol obtained in the method of chemical-mechanical depolymerizing polyester may be esterified and polycondensed, preparing the recycled polyester.

The esterification is a reaction between a dibasic acid and alkylene glycol which are recovered monomers, for example, terephthalic acid and ethylene glycol. This reaction may proceed without a catalyst but may also be performed under a catalyst of an alkaline-earth metal compound such as magnesium, calcium, and the like, a metal compound such as titanium, zinc, manganese, and the like, etc.

Subsequently, the products from the esterification process are polycondensed, preparing a recycled polyester resin. Herein, either solution polymerization or solid polymerization may be used.

In addition, after the solid polymerization, a compound capable of promoting a water treatment and/or crystallization may be added to adjust quality, if needed, and in addition, a polycondensation catalyst or a stabilizer may be added at the beginning or during the polycondensation process.

Herein, the water treatment may be, for example, carried out by contacting the recycled polyester having resin solid particles with water, aqueous vapor, aqueous vapor-containing inert gas, aqueous vapor-containing air, etc. The compound promoting the crystallization may be, for example, a polyolefin-based thermoplastic resin such as polyhexamethylene terephthalate, an inorganic compound, a higher aliphatic compound, a polyether-based compound, polypropylene, polyethylene, and the like, and these compounds may be added in an amount of 1 ppm to 100 ppm to the recycled polyester resin.

**The** polycondensation catalyst may include a compound such as germanium, antimony, titanium, aluminum, and the like. An amount of the polycondensation catalyst may be 2 ppm to 800 ppm, or for example 4 ppm to 500 ppm, as a weight of the catalytic metal element relative to the total weight of the dibasic acid component.

**The** recycled polyester may also include a phosphoric acid ester such as trimethyl phosphate, triethyl phosphate, triphenyl phosphate, and triethyl phosphonoacetate, a phosphorous acid ester such as triphenyl phosphite and trisodium phosphite, or a phosphorus compound such as methyl phosphate, dibutyl phosphate, monobutyl phosphate, phosphoric acid, phosphorous acid, hypophosphorous acid, and polyphosphoric acid, as a stabilizer. An amount of stabilizer may be less than or equal to 1000 ppm, for example less than or equal to 500 ppm, or less than or equal to 300 ppm, as weight of the phosphorus element in the stabilizer relative to the total weight of the recycled polyester.

On the other hand, as described above, since the method for chemical-mechanical depolymerizing polyester according to one embodiment pulverizes polyester and simultaneously depolymerizes it by heat generated therefrom, recycled polyester produced by using dibasic acid and alkylene glycol obtained therefrom may have a color with L of greater than or equal to 45, for example, greater than or equal to 60, or 65 to 95.

The **L,** a, and b color system is used internationally as a standard for color evaluation of polyester. These color values are one of the color systems to standardize color measurements and describe perceptible colors and color differences. In this system, L is a lightness factor, and a and b are color measurements. Herein, L is a numerical factor representing brightness and very important in manufacturing fibers, fabrics, or clothes. In addition, positive b means a yellowish coloration, negative b means a bluish discoloration, positive a means a reddish coloration, and negative a means a greenish coloration.

The **L,** a, and b values are defined in Korean Industrial Standards (KS) related to color measurements of KS A 0061, 0063, 0064, 0065, 0066, 0067, 0084, 0085, 0089, 0114, etc., and for example, may be obtained by using 50 g of a polyester resin, removing moisture therefrom in the air, and putting it in a colorimeter, SA-2000, to measure its color 10 times and calculate an average thereof.

The L value of the recycled polyester has relationship with purity of the recycled dibasic acid and alkylene glycol. For example, the lower the L value, the more impurities or matting agents such as titanium oxide (TiO₂) therein. When the L value is less than 45, the amount of the impurities is too large, which may color the obtained polyester after polymerization or fiberization, or generate many side reactions during the polymerization, so the polyester may not be used as a high value-added material but is mainly applied to the same use as mechanically recycled polyester. On the other hand, when the L value is greater than 95, which is difficult to reach even by existing virgin materials, the recycled polyester may achieve this L value by decolorization, addition of a purification process or an increase in residence time, etc., which may increase a unit production volume and a process cost and turn out to be less economical.

### [Mode for Invention]

Hereinafter, specific examples of the invention are presented. However, the examples described below are only intended to specifically illustrate or describe the invention, and this should not limit the scope of the invention.

### [Preparation Example: Mechanical-chemical depolymerization of polyester]

### (Example 1)

1 kg of waste polyethylene terephthalate (waste PET) and 416.3 g of sodium hydroxide (NaOH) are added to a pulverizing reactor.

The pulverizing reactor is internally substituted with nitrogen (N₂) to suppress a side reaction after adding the raw materials and perform a reaction during the entire process under a nitrogen (N₂) atmosphere.

Subsequently, a disk of pulverizing reactor is rotated at 1,200 rpm to generate heat by friction and grinding and depolymerize the waste PET into terephthalic acid (TPA) salt and ethylene glycol (EG). At the same time, the heat of 240 °C generated by the friction and grinding is used to recover EG by gasifying it

The gasified EG is under a normal pressure condition, and nitrogen (N₂) is injected thereinto during the process to liquefy and recover it in a condenser with the gas flow.

After separating EG, TPA salt powder is discharged from the pulverizing reactor.

The TPA salt is dissolved in water and then, purified and precipitated to recover terephthalic acid (TPA).

### (Example 2)

1 kg of waste polyethylene terephthalate (waste PET) is added to a pulverizing reactor.

Subsequently, while a disk of the pulverizing reactor is rotated at 500 rpm, 832.6 g of a sodium hydroxide aqueous solution (NaOH 50 wt%) is uniformly added thereto by spraying.

The pulverizing reactor is internally substituted with nitrogen (N₂) to suppressing a side reaction after adding the raw materials and perform a reaction during the entire process under a nitrogen (N₂) atmosphere.

Subsequently, the disk of pulverizing reactor is rotated at 1,200 rpm to generate heat by friction and grinding and depolymerize the waste PET into terephthalic acid (TPA) salt and ethylene glycol (EG). At the same time, the heat of 240 °C generated by the friction and grinding is used to recover EG and water (H₂O) by gasifying them. The gasified EG is under a normal pressure condition, and nitrogen (N₂) is injected thereinto to liquefy and recovering it in a condenser with the gas flow during the process.

After separating EG, TPA salt powder is discharged from the pulverizing reactor.

The TPA salt is dissolved in water and then, purified and precipitated to recover terephthalic acid (TPA).

### (Comparative Example 1)

Waste polyethylene terephthalate (waste PET) is pulverized into a size of less than 10 mm.

1 kg of the pulverized waste polyethylene terephthalate (PET) and 416.3 g of sodium hydroxide (NaOH) are dissolved in 6 kg of ethylene glycol (EG) and then, supplied to a depolymerization reactor and depolymerized at 180 °C for 60 minutes, while stirring with an agitator.

Subsequently, the depolymerization products, terephthalic acid (TPA) salt and ethylene glycol (EG), are separated into a solid and a liquid by using a decanter device, wherein the liquid-phased ethylene glycol (EG) is recovered through distillation to remove impurities, and the solid-phased terephthalic acid (TPA) salt is dissolved in water to remove impurities.

Then, sodium hydroxide (NaOH) is added to the terephthalic acid (TPA) salt aqueous solution having no impurities to reduce it to pH 3.0 or less, recovering terephthalic acid (TPA).

### [Experimental example: Physical properties of terephthalic acid]

The terephthalic acids prepared according to Examples 1 and 2 and Comparative Example 1 are measured with respect to physical properties, and the results are shown in Table 1.

### 1) ΔY (delta Y)

Each of the recycled terephthalic acid (rTPA) samples is taken in a small amount into a beaker and dissolved in an ammonia aqueous solution of NH₄OH at a concentration of 2.8% for 1 hour or more, and when the dissolution is completed, after passing the solution through a glass filter paper (a pore size: 0.4 µm), the filter paper is dried at 50 °C for 1 hour and left to cool. The cooled filter paper is placed on a measuring position by using a color spectrometer to measure a Y value (A) and then, passed by a blank test solution (a solution containing NH₄OH alone without the rTPA sample) and dried to measure another Y value (B) as a reference, which are used to calculate ΔY= A- B.

### 2) Alkali Transmittance (%)

Each of the rTPA samples is taken in a small amount into a beaker and then, stirred with a 2 N KOH an aqueous solution at 300 rpm to 500 rpm for 1 hour until the rTPA is completely dissolved, and the solution is three times repeatedly measured with respect to transmittance at 40 nm by adding a 2 N KOH solution alone in an empty cell of a UV-VIS spectrometer to correct the transmittance to 100% and taking the rTPA completely dissolved in the 2 N KOH, which are used to calculate a transmittance average.

### 3) Forced heat resistance (Heat resistance transmittance, %)

Each of the rTPA samples is taken in a small amount, added to a forced heat-resistant test tube, and after placing the test tube in a block heater, heating it at 280 °C for 3 hours, and leaving it to cool, the heat-treated sample is taken in a small amount into a 100 ml beaker and then, stirred with a 2 N KOH aqueous solution at 300 rpm to 500 rpm for 1 hour until it is completely dissolved therein. The dissolved sample is transported to a centrifuge tube and centrifuged at 3000 rpm for 15 minutes to take a supernatant, which is three times repeatedly measured with respect to transmittance at 40 nm by adding a 2 N KOH solution alone to an empty cell of a UV-VIS spectrometer to correct the transmittance to 100% and taking the rTPA completely dissolved in the 2 N KOH, which are used to calculate a transmittance average.

### 4) Acid value

Each of the rTPA samples is taken in a small amount (A g) into a beaker, and pyridine and pure water (DIW) are added to the corresponding beaker and then, stirred at 300 rpm to 500 rpm for 1 hour until the sample is completely dissolved, and after applying three drops of the pyridine, pure water, phenolphthalein indicators to an empty test beaker, the solution is titrated, while stirring by using an automatic burette device (Dosimat) equipped with a 0.5 N (Factor=1) NaOH standard solution. Herein, an end point, when a color change occurs to purple from colorless, is checked to measure a consumed amount of 0.5 N NaOH (b ml), and an end point of the beaker in which the rTPA sample is dissolved is also checked to measure a consumed amount of 0.5 N NaOH (a ml) in the same method, which are used to calculate an acid value (KOHmg/g) = (a-b)ml x F x 28.06 ÷ Ag.

**(Table 1)**

| | Forced heat resistance | Alkali transmittance | Acid value | ΔY |
|---|---|---|---|---|
| Example 1 | 80 | 83.6 | 673.3 | 33 |
| Example 2 | 82.5 | 91.1 | 675.1 | 18.1 |
| Comparative Example 1 | 82.2 | 81.9 | 672.8 | 46.5 |

Referring to Table 1, Examples 1 and 2, compared with Comparative Example 1, exhibit similar characteristics, which confirms that the examples are much more economical methods compared with the comparative example by using no solvent and recovering EG decomposed by the depolymerizing process.

Although the exemplified embodiments of the present invention have been described above, but the present invention is not limited thereto but may be implemented with various modifications within the scope of the claims, which also belong to the present invention.

### [Industrial Applicability]

The present disclosure relates to a method for chemical-mechanical depolymerizing polyester, which requires no additional processes such as washing, pulverizing, processing, or the like before adding a waste collected from industries or common households before adding it in a chemical process, is not limited to types of the waste, and needs neither a large amount of additional solvent nor a separate heat source for a reaction to proceeds with a simple device configuration and a low investment cost and also, is environmentally friendly due to no treatment and disposal of the solvent.

## Claims

1. A method for chemical-mechanical depolymerizing polyester, comprising
pulverizing polyester in the presence of a decomposer, generating heat, and depolymerizing the polyester into a dibasic acid and an alkylene glycol, and
separating the alkylene glycol by vaporizing it using the heat.

2. The method for chemical-mechanical depolymerizing polyester of claim 1, wherein
the depolymerizing is performed in a pulverizing reactor including a rotating disk.

3. The method for chemical-mechanical depolymerizing polyester of claim 2, wherein
the pulverizing reactor further includes blades attached to the disk.

4. The method for chemical-mechanical depolymerizing polyester of claim 2, wherein
the pulverizing reactor further includes baffles on the inner wall.

5. The method for chemical-mechanical depolymerizing polyester of claim 1, wherein
the heat includes pulverizing heat generated by pulverizing the polyester, frictional heat generated by friction of the polyester with the pulverizing reactor, or both.

6. The method for chemical-mechanical depolymerizing polyester of claim 1, wherein
the vaporizing and separating of the alkylene glycol are performed by additionally supplying heat from the outside.

7. The method for chemical-mechanical depolymerizing polyester of claim 1, wherein
the depolymerizing is a solvent-free reaction that proceeds without adding a solvent.

8. The method for chemical-mechanical depolymerizing polyester of claim 1, wherein
the method for chemical-mechanical depolymerizing polyester does not further include a process of pulverizing, crushing, or cutting the polyester before or after the depolymerizing.

9. The method for chemical-mechanical depolymerizing polyester of claim 2, wherein
the depolymerizing is performed by rotating the disk at 500 rpm to 5000 rpm.

10. The method for chemical-mechanical depolymerizing polyester of claim 2, wherein
the temperature inside the pulverizing reactor is set to 130 °C to 300 °C by the heat.

11. The method for chemical-mechanical depolymerizing polyester of claim 1, wherein
the depolymerizing is performed for 5 to 120 minutes.

12. The method for chemical-mechanical depolymerizing polyester of claim 1, wherein
the decomposer includes an alkali, an acid, a salt thereof, a monoalcohol, a polyhydric alcohol, or a mixture thereof.

13. The method for chemical-mechanical depolymerizing polyester of claim 12, wherein
the alkali includes sodium hydroxide, potassium hydroxide, lithium hydroxide, ammonia, or a mixture thereof,
the acid includes hydrochloric acid, nitric acid, sulfuric acid, carbonic acid, phosphoric acid, acetic acid, hypochlorous acid (HClO), or a mixture thereof,
the salt includes a carbonate, a bicarbonate, a phosphate, a sulfate, a sulfite, a nitrate, a silicate, a hypochlorite, a formate, an acetate, a citrate, an oxalate, or a mixture thereof,
the monoalcohol includes methanol, ethanol, propanol, butanol, or a mixture thereof, and
the polyhydric alcohol includes ethylene glycol, n-propylene glycol, isopropylene glycol, diethylene glycol, polyethylene glycol, triethylene glycol, dipropylene glycol, 1,3-butanediol, 1,4-butanediol, glycerin, benzyl alcohol, polypropylene glycol, pentaerythritol, trimethylolpropane, or a mixture thereof.

14. The method for chemical-mechanical depolymerizing polyester of claim 1, wherein
the decomposer is in a solid state or an aqueous solution state.

15. The method for chemical-mechanical depolymerizing polyester of claim 1, wherein
the decomposer is added in an amount of 0.75 to 3.0 moles per 1.0 mole of dibasic acid included in the polyester.

16. The method for chemical-mechanical depolymerizing polyester of claim 1, wherein
in the method for chemical-mechanical depolymerizing polyester, the alkylene glycol is separated and the remaining dibasic acid is obtained in the form of a solid salt.

17. The method for chemical-mechanical depolymerizing polyester of claim 16, wherein
the method further includes additionally separating remaining dibasic acid from a solid dibasic acid salt.

18. The method for chemical-mechanical depolymerizing polyester of claim 16, wherein
in the method for chemical-mechanical depolymerizing polyester,
the solid-phase dibasic acid salt is dissolved in water,
an aqueous solution of the dibasic acid salt is neutralized with acid to precipitate dibasic acid crystals, and
the dibasic acid crystals are separated from the precipitated product by solid-liquid separation.

19. The method for chemical-mechanical depolymerizing polyester of claim 18, wherein
the method for chemical-mechanical depolymerizing polyester includes removing impurities from the aqueous solution of the dibasic acid salt.

20. The method for chemical-mechanical depolymerizing polyester of claim 18, wherein
the method for chemical-mechanical depolymerizing polyester includes recrystallizing the dibasic acid crystal.

21. A composition for polymerizing a recycled polyester, comprising a dibasic acid and an alkylene glycol, obtained by the method for chemical-mechanical depolymerizing polyester according to claim 1.

22. A recycled polyester produced using a dibasic acid and an alkylene glycol, obtained by the method for chemical-mechanical depolymerizing polyester according to claim 1.

23. The recycled polyester of claim 22, wherein
a color of the recycled polyester has an L value of greater than or equal to 45.
